Europäisches Patentamt

European Patent Office

Office européen des brevets

(11) Numéro de publication: **0 059 148**
**B1**

(12) **FASCICULE DE BREVET EUROPEEN**

(45) Date de publication du fascicule du brevet: **29.05.85**

(51) Int. Cl.⁴: **A 61 K 33/24,** A 61 K 33/00

(21) Numéro de dépôt: **82400298.4**

(22) Date de dépôt: **19.02.82**

(54) Le chlorure de gallium, nouveau médicament anti-cancéreux.

(30) Priorité: **20.02.81 FR 8103355**

(43) Date de publication de la demande:
**01.09.82 Bulletin 82/35**

(45) Mention de la délivrance du brevet:
**29.05.85 Bulletin 85/22**

(84) Etats contractants désignés:
**AT BE CH DE GB IT LI LU NL SE**

(56) Documents cités:
**ARZNEIMITTELFORSCHUNG, vol. 25, mai 1975, Editio Cantor, AULENDORF/WÜRTT. (DE), L.J. ANGHILERI: "On the antitumor activity of gallium and lanthanides", pages 793-795 CHEMICAL ABSTRACTS, vol. 75, no. 13, 27 septembre 1971, abrégé 86912g, page 228, COLUMBUS OHIO (US)**

Le dossier contient des informations techniques présentées postérieurement au dépôt de la demande et ne figurant pas dans le présent fascicule.

(73) Titulaire: **LES LABORATOIRES MERAM Société Anonyme dite:**
**4 Bld Malesherbes**
**F-75008 Paris (FR)**

(72) Inventeur: **Collery, Philippe**
**63, rue Jules Blondeau**
**F-51160 Ay (FR)**

(74) Mandataire: **Gillard, Marie-Louise et al**
**Cabinet Beau de Loménie 55, Rue d'Amsterdam**
**F-75008 Paris (FR)**

(56) Documents cités:
**CHEMICAL ABSTRACTS, vol. 83, no. 11, 15 septembre 1975, abrégé 91092j, page 67, COLUMBUS OHIO (US)**
**Cancer Chemother. Reports 1975, 59/3), pp 599-610**

Courier Press, Leamington Spa, England.

## Description

L'invention a pour objet l'utilisation du chlorure de gallium de formule Cl₃Ga, comme médicament pour le traitement des tumeurs malignes.

On sait que le magnésium est nécessaire à la croissance tumorale et que la concentration en magnésium est élevée dans les tumeurs malignes. Pour ralentir la croissance tumorale, il faudrait donc avoir recours à un inhibiteur compétitif du magnésium qui prendrait sa place dans la cellule maligne sans remplir sa fonction. On a déjà observé que le gallium était capable de déplacer le magnésium de la cellule (Anghileri L. J., Strahlentherapie 1973, *146*, 359—366). Certains auteurs ont également rapporté que les sels de gallium pouvaient avoir une activité anti-tumorale chez l'animal, mais cette activité n'a pas été retrouvée par la suite et des essais d'utilisation du nitrate de gallium en thérapeutique humaine n'ont pas été poursuivis, en raison des résultats mineurs observés, et de la toxicité rénale trop importante du produit.

L'Article de Anghileri dans Arzneimittel-Forschung (vol. 25 p. 793—795 — 1975) est relatif à la comparaison des effets du gallium avec ceux des lanthanides, sur des tumeurs expérimentales; dans ces travaux, le chlorure de gallium et les lanthanides sont administrés par voie intra-péritonéale chex des animaux d'eassais porteurs de tumeurs expérimentales transplantées par voie sous cutanée.

L'Article de Adamson et al. dans Cancer Chemotherapy reports (partie 1 vol. 59 n° 3 mai/juin 1975, p. 599—610) est relatif à une étude des activités antitumorales du nitrate de gallium et d'autres sels de métaux du groupe IIIA. Il est indiqué dans cet article que le nitrate de gallium est particulièrement actif sur les tumeurs solides transplantées par voie sous cutanée et que ce sel a une utilité potentielle dans le traitement des tumeurs solides chez l'homme. On notera que dans les essais rapportés dans cet article, le nitrate de gallium est administré par voie intra-péritonéale ou par voie sous cutanée. Les résultats du tableau 4 de cet article montrent que le nitrate de gallium provoque des troubles rénaux chez la souris et le rat. Le chlorure de gallium est également mentionné dans cet article (page 605); cependant, les commentaires au sujet de ce sel n'incitent pas l'homme de l'art à l'utiliser pour le traitement de tumeurs.

On a trouvé, selon l'invention, que le chlorure de gallium peut être utilisé par voie orale avec de bons résultats et sans inconvénients majeurs, dans le traitement des tumeurs malignes chez l'homme.

La présente invention a donc pour objet des compositions pharmaceutiques, pour le traitement des tumeurs malignes, contenant à titre d'ingrédient actif, du chlorure de gallium, de formule GaCl₃, associé à un véhicule pharmaceutique spécifiquement adapté pour l'administration par voie orale.

L'invention concerne également un procédé pour l'obtention de telles compositions. Elle concerne également l'utilisation du chlorure de gallium pour la préparation d'un médicament pour le traitement des tumeurs malignes, ledit médicament étant sous la forme d'une composition pharmaceutique spécifiquement adaptée pour l'administration orale.

L'effet anti-tumoral de chlorure de gallium a d'abord été étudié chez des chiennes présentant des tumeurs malignes spontanées mammaires. Une thérapeutique par le chlorure de gallium a été effectuée chez des chiennes présentant un cancer mammaire (adénocarcinome tubulo-papillaire ou tumeur mixte); le traitement commençait, soit au stade de la tumeur primaire (4 chiennes), soit lors d'une récidive après une exérèse chirurgicale (10 chiennes).

Le chlorure de gallium était administré sous forme d'ampoules buvables contenant 1 mg ou 10 mg par ml de produit actif, à la dose de 1 mg/kg chez les premières chiennes; cette dose fut rapidement augmentée à 5 mg/kg dès que la tolérance s'est avérée bonne, notamment du point de vue digestif, et la durée du traitement fut de deux mois.

Les résultats furent les suivants:

1) Chez les 4 chiennes traitées au stade de tumeur primaire, on a constaté:
— un mort le 7e jour dans un cas;
— une régression de la tumeur dans les trois autres cas.

Dans deux cas le traitement fut poursuivi pendant deux mois, la tumeur régressant de 10×7 cm à 8,5×7 cm dans l'un des cas, et de 1,5×1,3 à 1,5×1,0 cm dans l'autre cas.

Dans le troisième cas, le traitement pouvait être poursuivi plus de six mois chez une très vieille chienne de 14 ans. Pendant deux mois, la posologie était de 5 mg/kg, puis le traitement fut stoppé pendant un mois à cause d'un dyspnée, puis repris à la posologie de 2,5 mg/kg pendant trois mois et demi, réduite à 1 mg/kg le mois suivant. La tumeur, qui avait doublé de taille pendant les quatre mois précédant le traitement, et atteignait 3,6×2 cm, n'évoluait pas pendant les deux premiers mois de traitement, augmentait de nouveau pendant le mois sans traitement, pour régresser après la reprise du traitement (3×2 cm) et devenir très dure.

2) Chez les 10 chiennes traitées au stade de la récidive, on constait:
— une mort à la troisième semaine de traitement, due à une métastase pulmonaire;
— trois rémissions complètes après deux mois de traitement, chez des chiennes dont la tumeur récidivante, avait, en début de traitement, une taille inférieure à 0,6 cm de diamètre;
— chez 5 chiennes, une régression partielle de la tumeur avec durcissement de celle-ci après trois mois de traitement.

Les biopsies effectuées en fin de traitement montrent que des cellules tumorales persistent, mais l'étude histologique suggère une augmentation de la substance fondamentale avec une raréfaction cellulaire;

— une persistance de la croissance de la tumeur chez une chienne traitée par 1 mg/kg de chlorure de gallium pendant un mois, posologie qui fut augmentée à 2,5 mg/kg pendant les quinze jours suivants, puis à 5 mg/kg pendant les quinze derniers jours.

L'efficacité de la thérapeutique par le chlorure de gallium peut n'apparaître qu'après plusieurs mois de traitement. C'est ce qui a été constaté à l'occasion du traitement de deux chiennes, atteintes de tumeurs mammaires initialement très évolutives (car il s'agissait pour ces deux chiennes d'une troisième récidive qui normalement entraîne le décès très rapidement, en quelques semaines).

Observation de la chienne OUD
. Posologie du chlorure de gallium, per os: 7,5 mg/kg du 15.4.1981 au 14.9.1981, puis 10 mg/kg.
. Histologie de cette troisième récidive: adénocarcinome (diagnostic par ponction à l'aiguille à biopsie)
. Taille de la tumeur:

> 5,- cm de diamètre le 15.4.1981
> 5,5×8,5 cm de diamètre le 26.5.1981
> 5,5×8,5 cm de diamètre le 25.6.1981
> 6,5×9,5 cm de diamètre le 14.9.1981
> 9,5×10 cm de diamètre le 14.10.1981

et ensuite *stabilisation tumorale* jusqu'au 28 décembre 1981 où la chienne meurt brutalement (bon état général les jours précédents) d'un infarctus du myocarde avec oedème aigu du poumon et défaillance cardiaque globale.
. A l'autopsie: pas de métastases hépatiques, ni pulmonaires. Un petit nodule de 1 cm dans la rate.
. Tolérance clinique et rénale: parfaite.

Observation de la chienne BEL
. Posologie du chlorure de gallium per os: 2,5 mg/kg du 27.2.1981 au 30.4.1981, puis 5 mg/kg jusqu'au 3.11.1981, puis 7 mg/kg.
. Histologie (comme chienne OUD...)
. Taille de la tumeur:

> 1,2 cm de diamètre le 27.2.1981
> 1,8 cm de diamètre le 1.4.1981
> 2,3 cm de diamètre le 30.4.1981
> 3,-×2,5 cm de diamètre le 25.5.1981
> 4,8×3,5 cm de diamètre le 2.7.1981
> 5,2×4,2 cm de diamètre le 30.7.1981
> 6,-×4,5 cm de diamètre le 1.9.1981

puis la croissance c'est nettement ralenti avec un changement évident de la consistance de la tumeur.

La tumeur a néanmoins continué de progresser:

> 6×4,5 cm de diamètre le 4.10.1981
> 7×5,5 cm de diamètre le 3.11.1981
> 8×6,- cm de diamètre le 3.12.1981
> 8×6,- cm de diamètre le 6.1.1982.

date à laquelle il est pratiqué l'exérèse chirurgicale de cette tumeur qui n'avait pratiquement pas grossie depuis le 3 novembre 1981.
A l'ouverture de la pièce d'exérèse: importante nécrose occupant la presque totalité de la tumeur.

. Tolérance clinique et rénale: parfaite. Radiographie pulmonaire du 5.1.1982: normale.

Le chlorure de gallium, à la dose de 5 mg/kg a donc donné de bons résultats dans le traitement des tumeurs mammaires de la chienne, à condition que la durée du traitement puisse être prolongée. La tolérance clinique et biologique est excellente. La régression de la tumeur reste faible et semble être davantage une rétraction de la tumeur qui devient très dure et apparaît comme une pierre, à l'exérèse. Un traitement chirurgical à ce stade semble alors recommandé. Grâce à cette stratégie thérapeutique, sept chiennes sur huit sont en vie et le temps de survie est maintenant significatif.

La toxicité aigüe du chlorure de gallium a été étudiée sur souris: la $DL_{50}$ per os est égale à 10,1 mmoles/kg (soit 1790 mg/kg) avec une limite fiducielle comprise entre 8,2 et 12,5 mmoles/kg (soit entre 1450 et 2209 mg/kg) pour p=0,05.

Les tumeurs mammaires de la chienne constituant un excellent modèle expérimental, et les résultats étant reproductibles chez l'homme, une expérimentation clinique a été entreprise chez des malades atteints de cancers dépassés.

ler malade
Madame B..., âgée de 35 ans, subit en octobre 1978 une ovariectomie bilatérale et une hystérectomie subtotale pour un cystadéno-carcinome bilatérale de l'ovaire. Une chimiothérapie post-opératoire (5-fluoro-uracile et endoxan) est ensuite réalisée jusqu'en avril 1979. A cette date, elle subit une deuxième intervention avec exérèse du col restant et omentectomie. Un envahissement épiploïque et ganglionnaire est découvert. Une cobaltothérapie est alors pratiquée du 5 novembre 1979 au février 1980 sur l'abdomen, le pelvis et les coupoles diaphragmatiques, puis une nouvelle chimiothérapie (hexaméthylmélamine) de août à octobre 1980. En octobre 1980 apparaît un volumineux oedème de toute la jame gauche et l'échotomographie du 27 octobre 1980 met en évidence une masse de 3,5 cm au niveau du cul de sac de Douglass. Cette masse est hétérogène, s'étend en latérovésical gauche, elle paraît à ce niveau infiltrer la paroi et mesure 4,4 sur 3 cm. Toute tentative de perfusion est vouée à l'échec de même qu'une dénudation veineuse. Un traitement par le chlorure de gallium est commencé le 1er novembre 1980 à la posologie de 300 mg par jour per os en trois prises de 100 mg. Après 2 mois de traitement ininterrompu, la bonne tolérance clinique et biologique est constatée. L'échotomographie du 9 décembre 1980 montre que la masse du cul de sac de Douglass est hétérogène, et conserve le même diamètre, mais la masse superficielle ne peut plus être mesurée car ses contours sont mal délimités et surtout l'interface vésicotumoral paraît très dense et donne un cône d'atténuation en arrière indiquant que le tissue d'infiltration est devenu un tissu scléreux. Une troisième échotomographie est réalisée le 7 janvier 1981: le volume de la masse du cul de sac de Douglass a nettement diminué de volume et s'est ovalisée. Fin janvier 1981, l'état clinique est très satisfaisant et la masse superficielle que l'on pouvait nettement palper en octobre 1980 a disparu, de sorte que l'on peut envisager une intervention chirurgicale, mais celle-ci est refusée par la malade. Le 20 mars 1981, une douleur dans le flanc gauche et l'épigastre survient soudainement avec une dilatation du colon gauche. Le traitement par le chlorure de gallium devait être interrompu du fait d'une intolérance digestive. Un état sub-occlusif s'installe les jours suivants, puis, après une brève amélioration, plusieurs épisodes sub-occlusifs surviennent et un ascite apparaît. Selon les chirurgiens, il n'y a aucune possibilité chirurgicale. L'état de santé s'aggrave très rapidement et la mort survient le 6 mai 1981.

2e malade

Monsieur DUP . . . Jean, âgé de 50 ans est suivi depuis mars 1980 pour une tumeur cutanée de $3\times2$ cm au niveau de la région axillaire gauche. L'exérèse chirurgicale effectuée en avril 1980 montre qu'il s'agit d'un épithélioma massif occupant le derme et l'hypoderme. Un bilan complet à la recherche de la localisation primitive est entrepris. Devant la négativité de ce bilan le diagnostic de tumeur primitive cutanée, développée aux dépens des annexes cutanées (tumeur eccrine) est retenu.

Le bilan d'extension pratiqué parallèlement ne montre pas de métastases hépatiques, ni pulmonaires. Les radiographies osseuses montrent en enfoncement du plateau supérieur de la 12e vertébre dorsale ($D_{12}$) qui est considéré comme métastatique d'autant qu'il existe à ce niveau des douleurs de type inflammatoire. Une irradiation de 30 γ rays est délivrée de $D_9$ à $C_4$ pendant la période du 5 au 20 mai 1980. Cette irradiation est complétée de $D_1$ à $D_7$ par 16,5 γ rays en 3 séances du 31 juillet 1980 au 4 août 1980 devant l'apparition de douleurs dorsales hautes et un affaissement du plateau de $D_6$.

L'évolution est marquée par la découverte fin juillet 1980 d'une adénopathie de $4\times3$ cm, axillaire gauche, dont la nature métastatique est confirmée par une exérèse chirurgicale: il s'agit d'une métastase massive, ganglionnaire d'un adénocarcinome relativement différencié et secrétant. Une cure de chimiothérapie associant Adriamycine (50 mg au jour 1), 5-fluoro-uracile (900 mg les jours 2 et 3) et Endoxan (900 mg le jour 4) est suivie chez ce malade éthylique d'une pancytopénie qui persistera plusieurs mois et ne permettra pas de renouveler cette thérapeutique.

En avril 1981 apparaissent à nouveau des douleurs osseuses, dorsales hautes, continues, quotidiennes, non soulagées par le repos, avec des images évocatrices de métastase au niveau du corps de $D_7$ et un foyer net hyperactif à ce niveau à la scintigraphie osseuse, amenant à pratiquer une nouvelle irradiation de 15 γ rays de $D_1$ à $D_7$ du 4 au 12 mai 1981.

En mai 1981, une nouvelle adénopathie axillaire gauche est constatée, arrondie, de 3 cm de diamètre. Le traitement par le chlorure de gallium est commencé le 20 mai 1981, par voie orale, à raison de 500 mg par jour jusqu'au 15 juin 1981, puis 300 mg par jour du 15 juin 18 août, puis 600 mg par jour jusqu'au 18 décembre 1981, et enfin en alternance 1 jour sur 2, 600 mg et 300 mg.

L'efficacité du traitement par le chlorure de gallium se traduit par la régression progressive de l'adénopathie à partir de fin juillet 1981. Le 23 juillet 1981, l'adénopathie est beaucoup plus dure. Elle mesure $1\times1,5$ cm le 12 novembre 1981, puis 1 cm de diamètre le 8 décembre 1981 et n'est plus perceptible le 6 danvier 1982. A la date du 6 janvier 1982:

— le traitement par le chlorure de gallium est toujours poursuivi et parfaitement bien toléré, sans troubles digestifs ni manifestations neurologiques, cardiaques ou cutanées;
— l'état général est bon, avec un appétit normal, un poids de 71 kg inchangé depuis le début du traitement;
— il persiste des douleurs osseuses, dorsales, mais de type mécanique, complètement calmées par le repos, avec une déminéralisation d'ensemble des vertébres, peut-être séquelles de la radiothérapie;
— biologiquement:
. le bilan inflammatoire (haptoglobine) est normal,
. la créatinine est normale: 88,50 µmol/l,
. l'hémogramme, le bilan hépatique, la calcémie, la phosphorémie sont normaux,
. quant aux taux de Mg globulaire, ils étaient compris entre 2,80 et 3,20 mmol/l avant le traitement par le chlorure de gallium et sont entre 2,40 et 2,80 mmol/l en cours de traitement, ce qui témoigne de l'efficacité du traitement. L'importance de la surveillance dex taux de Mg globulaire est par ailleurs bien illustrée par les deux observations suivantes (3e et 4e malades).

3e malade

Monsieur MOR . . . Roger, âgé de 73 ans subit en avril 1981 une exérèse chirurgicale d'un mélanome malin du membre inférieur gauche. Il s'agit d'un mélanome nodulaire de $1,2\times1$ cm, Clark IV, avec Breslow supérieur à 3 mm et un index mitotique moyen. Il existe une formation adjacente de 1,2 sur 0,9 cm et 3 métastases cutanées sur le même membre. Il existe enfin un envahissement ganglionnaire homolatéral massif mais pas d'envahissement controlatéral. Il n'y a pas de métastases pulmonaires et hépatiques décelables mais le pronostic est d'emblée très défavorable.

Le traitement part le chlorure de gallium est instauré le 15 mai 1981, à la posologie de 600 mg/24 heures jusqu'au 20 septembre 1981, en association avec une héparinothérapie à mini-doses. Cette héparinothérapie avait été prescrite dans le cadre de la prévention d'un phlébite ou d'une embolie pulmonaire à la suite de l'intervention chirurgicale d'avril 1981 et n'avait pas été interrompue pour ne pas provoquer le phénomène de flambée tumorale. [Calciparine (sel de calcium d'héparine) 0,2 ml, 3 frois par jour en sous-cutané]. Jusqu'à cette date l'état général reste satisfaisant, avec bon appétit et poids stable de 70 kg. Les taux de magnésium globulaire sont remarquablement stables et à des taux compris entre 2,05 et 2,30 mmol/l. Le 20 septembre 1981, le traitement adjuvant par la Calciparine est interrompu pendant 3 jours pour l'exérèse chirurgicale d'un nodule métastatique souscutané de la cuisse gauche. Comme nous l'avons à plusieurs reprises constaté, l'interruption, même brève, de l'ordre de 48 heures à 72 heures, de la Calciparine, est suivie très rapidement d'une croissance tumorale rapide et insensible à toute thérapeutique. Ce fut le cas de M. MOR . . . Roger dont l'état général s'est ensuite rapidement dégradé, avec apparition d'une hépatomégalie métastatique dont le volume a rapidement augmenté malgré l'augmentation de la posologie du chlorure de gallium à 800 mg/24 heures à partir du 29 septembre 1981. Cette posologie a été bien tolérée jusqu'au 27 novembre 1981, mais l'anorexie presque totale à partir de cette date a entraîné l'arrêt de la thérapeutique. Jusqu'au 27 nombre 1981, la fonction rénale et l'hémogramme sont restés normaux. C'est à partir du 20 septembre 1981, date de l'arrêt de lhéparino-thérapie que l'on a constaté une augmentation mette (atteignant 2,96 mmol/l le 6 octobre 1981) des taux de magnésium globulaire.

4e malade

Monsieur HOT . . . Roland, âgé de 62 ans, est opéré le 14 mai 191 d'un adénocarcinome colique. Il s'agit d'une tumeur de l'angle droit, ulcérée et infiltrante, presque circonférentielle, étendue sur une hauteur de 4 cm, atteignant tous les plans de la paroi colique et diffusant dans le tissu adipeux avec une atteinte des ganglions du pédicule colique supérieur droit (avec dépassement capsulaire), de deux ganglions du mésocôlon transverse, un nodule tumoral au sein du tissu adipeux du nésocôlon descendant et une atteinte du grêle distal. Il est réalisé une colectomie presque totale, laissant 20 cm de sigmoïde et une résection du grêle sur 15 cm de long. Les suites opératoires sont satisfaisantes et le traitement par le chlorure de gallium est commencé, par voie orale, le 29 mai 1981 à la posologie de 500 mg/24 heures, jusqu'au ler juillet 1981, puis poursuivi à 300 mg/24 heures. Pendant 4 mois l'état général va rester très satisfaisant et les taux de Mg globulaire et d'antigène carcinoembryonnaire restent normaux. Le 15 octobre 1981 apparaissent des douleurs abdominales qui vont aller en s'accentuant et les taux de magnésium globulaire vont augmenter à 3,0 mmol/l et l'antigène carcinoembryonnaire à 9 ng/ml (puis à 97 ng/ml le 26 novembre 1981). Une laporotomie exploratrice est pratiquée le 12 novembre 1981 retrouvant un bloc iléo-mésentérique indisséquable et des granulations hépatiques réparties en grains de riz sur l'ensemble des deux lobes du foie. L'état général va continuer ensuite à se dégrader, un ictère apparaît et le décès survient le 7 janvier 1982. Chez ce malade, la thérapeutique par le chlorure de gallium poursuivie jusqu'au 10 novembre 1981 a été insuffisante à la posologie de 300 ng par jour pour prévenir une récidive. La tolérance a été parfaite cliniquement et biologiquement (sans atteinte rénale en particulier) et la corrélation entre l'état clinique, les taux d'antigène carcinoembryonnaire et de magnésium globulaire a pû être soulignée.

5e malade

Monsieur DRU . . . Michel, âgé de 68 ans: le diagnostic de carcinome épidermoïde différencié, mature et infiltrant de l'oesophage a été porté le 3 septembre 1981. Il s'agit d'une volumineuse tumeur, étendue de 23 à 32 cm, des arcades dentaires (selon les données de l'endoscopie) avec un envahissement de la face postérieure de l'origine de la bronche souche gauche et de la carène. A la bronchoscopie du 8 septembre 1981, il existe un bourgeon tumoral, nécrotique, obstruant aux 2/3 la lumière de la bronche.

La thérapeutique par le chlorure de gallium est commencée le 21 septembre 1981 à la posologie de 800 mg/24 heures par voie orale.

Une nouvelle bronchoscopie est pratiquée le 13 octobre 1981, montrant la même obstruction bronchique. L'existence d'une stase bronchique sous-jacente importante et d'accès dyspnéiques vespéraux quotidiens amène à traiter cette tumeur bronchique par le laser le 20 octobre 1981. Dans les suites de cet acte des troubles de la déglutition sont notés et une fistule oeso-bronchique est mise en évidence par un transit à la gastrographine. L'alimentation est interrompue par voie orale, de même que le traitement par le chlorure de gallium et après une alimentation parentérale, une prothèse de célestin est mise en place par voie endoscopique le 3 novembre 1981. Les suites de la pose de la prothèse sont marquées par l'apparition d'un syndrôme infectieux bronchique qui va entraîner le décès du malade le 18 novembre 1981. La thérapeutique par le chlorure de gallium n'a pû être poursuivie correctement que pendant un mois mais à la posologie de 800 mg/24 heures et pendant toute cette période, la thérapeutique a été parfaitement tolérée, tant du point de vue clinique que biologique, sans atteinte rénale, ni hépatique, ni perturbations de l'hémogramme.

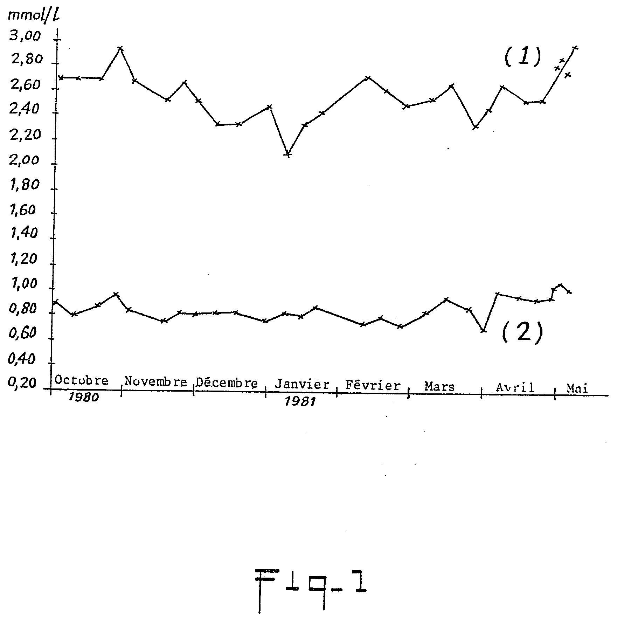

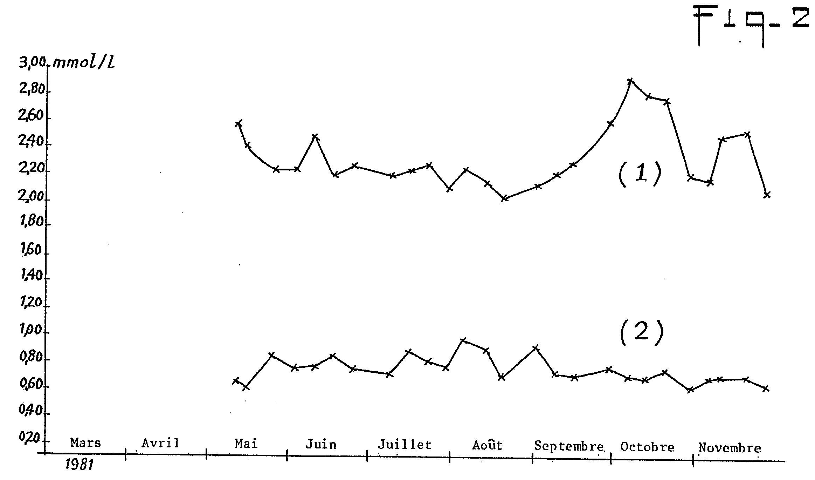

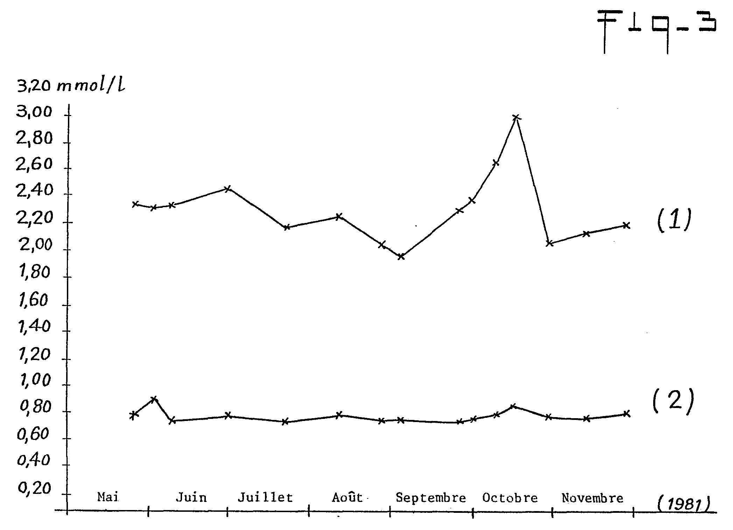

Les figures 1, 2 et 3 représentent les courbes des taux de Mg globulaire (1) et de Mg plasmatique (2) en fonction du temps au cours du traitement par le chlorure de gallium (le point de départ des courbes correspondant à la première administration) respectivement des trois malades, Mme BID...Marie-François (lère malade), M. MOR...Roger (3e malade) et M. HOT...Roland (4e malade).

Ces courbes montrent que les taux de magnésium globulaire diminuent sous l'effet du traitement par le chlorure de gallium quand celui-ci est efficace. Ils s'élèvent à nouveau en cas de reprise évolutive du processus tumoral, en particulier en cas d'arrêt du traitement.

Pour bien suivre les taux de Mg globulaire, les dosages doivent être fréquents et répétés, environ une fois par semaine. Il existe en effet des variations cycliques des taux de Mg globulaire avec une courbe d'aspect sinusoïdal, et la période de ces cycles, de même que l'importance des taux de Mg globulaire sont fonction du potentiel évolutif de la tumeur maligne (P. Collery et col. "Role of Magnesium in the Development of Cancer", Trace Substances in Environmental Health. XII, 1978 — A Symposium — University of Missouri — Columbia). L'aspect de cette courbe est modifié comme indiqué plus haut par la thérapeutique anti-tumorale par le chlorure de gallium.

Le chlorure de gallium peut donc constituer un médicament utile dans le traitement des tumeurs malignes, notamment des voies génitales. Il est administré par voie orale, à des doses pouvant aller de 200 mg à 1 g par jour selon les cas, le traitement devant durer au moins deux mois. La surveillance des taux de magnésium globulaire peut servir à juger de l'efficacité du traitement et à déterminer la date de la fin de celui-ci. Le principe actif peut être associé à tout véhicule classique spécifiquement adapté pour l'administration orale et conditionné sous toutes les formes administrables par voie orale, notamment sous forme d'ampoules buvables qui contiennent 100 à 500 mg de chlorure de gallium.

On donne ci-dessous, à titre d'exemple, la composition d'ampoules de 10 ml, dosées à 100 mg de produit actif.

| | |
|---|---|
| Chlorure de gallium | 0,10 g |
| Concentré d'orange 1/5 | 1,875 g |
| Essence de mandarine | 0,125 g |
| p-hydroxybenzoate de méthyle sodé | 0,00225 g |
| p-hydroxybenzoate de propyle sodé | 0,00025 g |
| Sucre cristallisé | 2 g |
| Eau distillée Q.S.P. 10 ml | |

**Revendications pour les Etats Contractants BE CH DE GB IT LI LU NL SE**

1. Compositions pharmaceutiques, pour le traitement des tumeurs malignes, contenant à titre d'ingrédient actif, du chlorure de gallium, de formule $Cl_3Ga$, associé à un véhicule pharmaceutique spécifiquement adapté pour l'administration par voie orale.

2. Compositions pharmaceutique selon la revendication 1, caractérisées en ce qu'elles contiennent le chlorure de gallium à la dose unitaire de 100 à 500 mg.

3. Compositions pharmaceutiques selon l'une des revendications 1 ou 2, caractérisées en ce qu'elles se présentent sous la forme d'ampoules buvables.

4. Ampoules buvables de chlorure de gallium, caractérisées en ce qu'elles contiennent 100 à 500 mg de chlorure de gallium associé à un véhicule pharmaceutique spécifiquement adapté pour l'administration par voie orale.

5. Procédé pour l'obtention des compositions pharmaceutiques selon l'une quelconque des revendications 1 à 4, caractérisé en ce qu'il consiste à associer du chlorure de gallium à tout véhicule classique spécifiquement adapté pour l'administration orale et à conditionner ledit mélange sous une forme appropriée pour l'administration orale.

6. Procédé selon la revendication 5, caractérisé en ce que le conditionnement est un conditionnement sous forme d'ampoules buvables.

7. Utilisation du chlorure de gallium pour la préparation d'un médicament pour le traitement des tumeurs malignes, ledit médicament étant sous la forme d'une composition pharmaceutique spécifiquement adaptée pour l'administration orale.

8. Utilisation selon la revendication 4, caractérisée en ce que le médicament contient le chlorure de gallium à la dose unitaire de 100 à 500 mg.

9. Utilisation selon l'une des revendications 4 ou 5, caractérisée en ce que le médicament est présenté sous la forme d'ampoules buvables.

**Revendications pour l'Etat Contractant AT**

1. Procédé pour la fabrication de compositions pharmaceutiques, pour le traitement des tumeurs malignes, contenant à titre d'ingrédient actif, du chlorure de gallium, de formule $GaCl_3$, associé à un véhicule pharmaceutique convenable pour une administration orale, caractérisé en ce qu'il consiste à associer du chlorure de gallium à tout véhicule classique spécifiquement adapté pour l'administration orale et à conditionner ledit mélange sous une forme appropriée pour l'administration orale.

2. Procédé selon la revendication 1, caractérisé en ce que le conditionnement est un conditionnement sous forme d'ampoules buvables.

3. Procédé selon l'une des revendications 1 ou 2, caractérisé en ce que le chlorure de gallium est associé audit véhicule en des quantités suffisantes pour fournir des doses unitaires de 100 à 500 mg de GaCl₃.

4. Utilisation du chlorure de gallium pour la préparation d'un médicament pour le traitement des tumeurs malignes, ledit médicament étant sous la forme d'une composition pharmaceutique spécifiquement adaptée pour l'administration orale.

5. Utilisation selon la revendication 4, caractérisée en ce que le médicament contient le chlorure de gallium à la dose unitaire de 100 à 500 mg, en association avec un véhicule pharmaceutique spécifiquement adapté pour l'administration par voie orale.

6. Utilisation selon l'une des revendications 4 ou 5, caractérisée en ce que le médicament est présenté sous la forme d'ampoules buvables.

**Patentansprüche für die Vertragsstaaten BE, CH, DE, GB, IT, LI, LU, NL, SE:**

1. Pharmazeutische Zusammensetzungen für die Behandlung von bösartigen Tumoren, welche als aktives Ingrediens Galliumchlorid der Formel GaCl₃ in Verbindung mit einem spezielle für Verabreichung auf oralem Weg adaptierten pharmazeutischen Träger enthalten.

2. Pharmazeutische Zusammensetzung nach Anspruch 1, dadurch gekennzeichnet, daß sie Galliumchlorid in einer Einheitsdosis von 100 bis 500 mg enthalten.

3. Pharmazeutische Zusammensetzung nach einem der Ansprüche 1 oder 2, dadurch gekennzeichnet, daß sie in Form von Trinkampullen vorliegen.

4. Galliumchlorid-Trinkampullen dadurch gekennzeichnet, daß sie 100 bis 500 mg Galliumchlorid in Verbindung mit einem speziell für Verabreichung auf oralem Weg adaptieren pharmazeutischen Träger enthalten.

5. Verfahren zur Herstellung von pharmazeutischen Zusammensetzungen nach einem der Ansprüche 1 bis 4, dadurch gekennzeichnet, daß es darin besteht, daß Galliumchlorid zu jedem herkömmlichen, speziell für orale Verabreichung adaptierten Träger zugemischt und die Mischung in für orale Verabreichung geeignete Form gebracht wird.

6. Verfahren nach Anspruch 5, dadurch gekennzeichnet, daß die geeignete Form aus Trinkampullen besteht.

7. Verwendung von Galliumchlorid zur Herstellung eines Medikaments für die Behandlung von bösartigen Tumoren, welches Medikament in Form einer speziell für orale Verabreichung adaptierten pharmazeutischen Zusammensetzung vorliegt.

8. Verwendung nach Anspruch 4, dadurch gekennzeichnet, daß das Medikament Galliumchlorid in einer Einheitsdosis von 100 bis 500 mg enthält.

9. Verwendung nach einem der Ansprüche 4 oder 5, dadurch gekennzeichnet, daß das Medikament in Form von Trinkampullen vorliegt.

**Patentansprüche für den Vertragsstaat AT:**

1. Verfahren zur Herstellung von pharmazeutischen Zusammensetzungen für die Behandlung von bösartigen Tumoren, welche als aktives Ingrediens Galliumchlorid der Formel GaCl₃ in Verbindung mit einem für orale Verabreichung geeigneten pharmazeutischen Träger enthalten, dadurch gekennzeichnet, daß es darin besteht, daß Galliumchlorid zu jedem herkömmlichen, speziell für orale Verabreichung adaptierten Träger zugemischt und die Mischung in für orale Verabreichung geeignete Form gebracht wird.

2. Verfahren nach Anspruch 1, dadurch gekennzeichnet, daß die geeignete Form aus Trinkampullen besteht.

3. Verfahren nach einem der Ansprüche 1 oder 2, dadurch gekennzeichnet, daß Galliumchlorid in zur Schaffung von Einheitsdosen von 100 bis 500 mg GaCl₃ ausreichenden Mengen zum Träger zugegeben wird.

4. Verwendung von Galliumchlorid zur Herstellung eines Medikaments für die Behandlung von bösartigen Tumoren, welches Medikament in Form einer speziell für orale Verabreichung adaptierten pharmazeutischen Zusammensetzung vorliegt.

5. Verwendung nach Anspruch 4, dadurch gekennzeichnet, daß das Medikament Galliumchlorid in einer Einheitsdosis von 100 bis 500 mg in Verbindung mit einem speziell für Verabreichung auf oralem Weg adaptierten pharmazeutischen Träger enthält.

6. Verwendung nach einem der Ansprüche 4 oder 5, dadurch gekennzeichnet, daß das Medikament in Form von Trinkampullen vorliegt.

**Claims for the Contracting States: BE, CH, DE, GB, IT, LI, LU, NL, SE**

1. Pharmaceutical compositions, for the treatment of malignant tumors, containing as active ingredient, gallium chloride, of formula Cl₃Ga, associated to a pharmaceutical carrier specifically adapted for the administration by oral route.

2. Pharmaceutical compositions according to claim 1, characterized in that they contain the gallium chloride in the unitary dosages of 100 to 500 mg.

3. Pharmaceutical composition according to one of claims 1 or 2, characterized in that they are in the form of drinkable ampoules.

4. Drinkable ampoules of gallium chloride, characterized in that they contain 100 to 500 mg of gallium chloride associated to a pharmaceutical carrier specifically adapted for administration by oral route.

5. Process for obtaining pharmaceutical compositions according to any one of claims 1 to 4, characterized in that it consists in associating gallium chloride to any conventional carrier specifically adapted for oral administration; and in packing said mixture in a suitable form of oral administration.

6. Process according to claim 5, characterized in that the packaging is a packaging in the form of drinkable ampoules.

7. Use of gallium chloride for the preparation of a drug for the treatment of malignant tumors, said drug being in the form of a pharmaceutical composition specifically adapted for oral administration.

8. Use according to claim 4, characterized in that the drug contains gallium chloride in the unitary dosages of 100 to 500 mg.

9. Use according to any one of claims 4 or 5,. characterized in that the drug is in the form of drinkable ampoules.

**Claims for the Contracting State AT**

1. Process for the manufacture of pharmaceutical compositions, for the treatment of malignant tumors, containing as active ingredient, gallium chloride, of formula $GaCl_3$ associated to a pharmaceutical carrier suitable for an oral administration, characterized in that it consists in associating gallium chloride to any conventional carrier specifically adapted for oral administration, and in packaging said mixture in a form suitable for oral administration.

2. Process according to claim 1, characterized in that the drug is in the form of drinkable ampoules.

3. Process according to any one of claims 1 or 2, characterized in that the gallium chloride is associated to said carrier in sufficient quantities to supply unitary dosages from 100 to 500 mg of $GaCl_3$.

4. Use of gallium chloride for the preparation of a drug for the treatment of malignant tumors, said drug being in the form of a pharmaceutical composition specifically adapted for oral administration.

5. Use according to claim 4, characterized in that the drug contains gallium chloride in the unitary dosages of 100 to 500 mg, in association with a pharmaceutical carrier specifically adapted for the administration of oral route.

6. Use according to any one of claims 4 or 5, characterized in that the drug is in drinkable ampoules form.

Fig-1

Fig-2

0 059 148

Fig-3

3,20 mmol/L

3,00
2,80
2,60
2,40
2,20
2,00
1,80
1,60
1,40
1,20
1,00
0,80
0,60
0,40
0,20

(1)

(2)

Mai    Juin    Juillet    Août    Septembre    Octobre    Novembre    (1981)